# EUROPEAN PATENT SPECIFICATION

(11) **EP 3 337 562 B1**
(45) Date of publication and mention of the grant of the patent: **24.07.2019**
(21) Application number: 16775007.4
(22) Date of filing: 15.08.2016
(51) Int. Cl.: A61N 5/10

(54) **RADIOACTIVE STENT**
RADIOAKTIVER STENT
ENDOPROTHÈSE VASCULAIRE RADIOACTIVE

(30) Priority: 17.08.2015 US 201562206236 P; 28.06.2016 US 201662355637 P
(43) Date of publication of application: 27.06.2018
(73) Proprietor: Boston Scientific Scimed Inc., Maple Grove, Minnesota 55311 (US)
(72) Inventor: HINGSTON, John A., Framingham, Massachusetts 01701 (US); CLERC, Claude O., Marlborough, Massachusetts 01752 (US)
(74) Representative: Vossius & Partner Patentanwälte Rechtsanwälte mbB
(86) International application number: PCT/US2016/047050
(87) International publication number: WO 2017/031065

(56) References cited:
- EP-A1- 0 433 011
- EP-A2- 0 819 446
- DE-A1- 19 913 978
- US-A1- 2010 137 673
- US-A1- 2015 190 654

## Description

### TECHNICAL FIELD

The present disclosure pertains to medical devices, and methods for preparing medical devices. More particularly, the present disclosure pertains to elongated intracorporeal medical devices including radioactive elements and methods for manufacturing such devices.

### BACKGROUND

Some cancers and neoplasms are easier to treat with radiation than others. Hard-to-reach neoplasms, such as those in the esophagus, intestines and other lumens, may be treated via brachytherapy so as to minimize radiation to adjacent, healthy tissue.

Brachytherapy delivers radiation to small tissue volumes while limiting exposure of healthy tissue. In this regard, the delivered radiation conforms more to the target than any other form of radiation, (including proton therapy) as less normal transient tissue is treated. It features placement of radiation sources, such as small radioactive particles or needles, near or within the target tissue, thus having the advantage over External Beam Radiation Therapy (EBRT) of being more focalized and less damaging to surrounding healthy tissue.

Brachytherapy is a common treatment for esophageal, prostate, and other cancers. Brachytherapy has been used to treat prostate cancer which has been practiced for more than half century. In this situation, very low activity material emitting a low energy is placed next to or within a tumor. Traditionally, these low emitting devices have mostly been left in place permanently except in extraordinary circumstances. It would be desirable to utilize radioactive material in conjunction with interventional medical devices when clinically appropriate, and/or it may be desirable to tailor the delivery of radioactive energy or radioactive sources according to clinical needs. For example, it may be advantageous to couple a radiation source with an expandable stent when clinically necessary and/or it may be advantageous to adjust the position and the activity of the radioactive source on a stent in response to changes in tumor shape and size, carrier position, and other relevant therapeutic factors.

US 2015/0190654 A1 discloses a medicament delivery vehicle comprising tubular members adapted to removably receive the medicament and an expandable stent adapted to receive the tubular members.

### BRIEF SUMMARY

A stent and a method of preparing a stent as recited in the independent claims are provided. The dependent claims define embodiments.

This disclosure provides design, material, and manufacturing method alternatives for medical devices. A stent includes an expandable framework and a plurality of tubular members disposed along the expandable framework. Each of the plurality of tubular members includes a lumen extending therein. The stent also include a coating, wherein the coating is applied directly to both the plurality of tubular members and the expandable framework.

Alternatively or additionally to any of the embodiments above, wherein the expandable framework includes one or more interstitial spaces, and wherein the coating is configured to span both the one or more interstitial spaces and the plurality of tubular members.

Alternatively or additionally to any of the embodiments above, wherein the coating attaches the plurality of tubular members to the expandable framework.

Alternatively or additionally to any of the embodiments above, wherein the plurality of tubular members and the expandable framework are embedded in the coating.

Alternatively or additionally to any of the embodiments above, wherein the expandable framework includes an inner surface, and wherein the plurality of tubular members are attached to the inner surface of the expandable framework.

Alternatively or additionally to any of the embodiments above, wherein the plurality of tubular members are helically wound around the inner surface of the expandable framework.

Alternatively or additionally to any of the embodiments above, further comprising a plurality of radioactive elements disposed within the lumen of one or more of the plurality of tubular members.

Alternatively or additionally to any of the embodiments above, further comprising one or more spacers positioned between two or more of the plurality of radioactive elements.

A method of preparing a stent, comprises:
positioning a plurality of tubular members on a mandrel, each of the plurality of tubular members including a lumen extending therein;
disposing an expandable framework over the tubular members and the mandrel; and
applying a coating to both the expandable framework and the tubular members.

Alternatively or additionally to any of the embodiments above, wherein positioning the plurality of tubular members on a mandrel includes winding the plurality of tubular members along grooves located on the surface of the mandrel.

Alternatively or additionally to any of the embodiments above, wherein the plurality of tubular members are helically wound around the surface of the mandrel.

Alternatively or additionally to any of the embodiments above, wherein the expandable framework includes one or more interstitial spaces, and wherein the coating is configured to span both the one or more interstitial spaces and the tubular members.

Alternatively or additionally to any of the embodiments above, wherein the coating attaches the tubular members to the expandable framework.

Alternatively or additionally to any of the embodiments above, wherein the expandable framework includes an inner surface, and wherein the plurality of tubular members are attached to the inner surface of the expandable framework

Alternatively or additionally to any of the embodiments above, further comprising positioning a plurality of radioactive elements within the lumen of one or more of the plurality of tubular members.

Alternatively or additionally to any of the embodiments above, further comprising one or more spacers positioned between two or more of the plurality of radioactive elements.

There is also disclosed an example mandrel, which comprises:
an elongate member having a first end portion, a second end portion and a body extending therebetween;
wherein the body includes a first helical groove extending along the length thereof;
wherein the first end portion includes a first securement region, the first securement region configured to secure a first end of a tubular member.

Alternatively or additionally to any of the embodiments above, wherein the second end portion includes a second securement region, the second securement region configured to secure a second end of the tubular member.

Alternatively or additionally to any of the embodiments above, wherein the first securement region includes a first opening extending through the first end portion and the second securement region includes a second opening extending through the second end portion, wherein the first and second openings are configured to permit the first and second ends of the tubular member to be secured therein.

Alternatively or additionally to any of the embodiments above, further comprising a second helical groove and a continuous raised land positioned between the first and second helical grooves, the continuous raised land extending along a length of the body from the first end portion to the second end portion.

The above summary of some embodiments is not intended to describe each disclosed embodiment or every implementation of the present disclosure. The Figures, and Detailed Description, which follow, more particularly exemplify these embodiments.

### BRIEF DESCRIPTION OF DRAWINGS

The disclosure may be more completely understood in consideration of the following detailed description in connection with the accompanying drawings, in which:
FIG. 1 is an example radioactive stent.
FIG. 2 illustrates a cross-sectional view of an example radioactive stent.
FIG. 3 illustrates an example mandrel used to prepare a radioactive stent.
FIG. 4A illustrates a cross-sectional view of an example radioactive stent.
FIG. 4B illustrates an alternative cross-sectional view of an example radioactive stent.
FIG. 5 is an end view of an example mandrel used to prepare a radioactive stent.
FIG. 6 is an end view of an example mandrel used to prepare a radioactive stent.
FIG. 7 illustrates an example method to prepare a radioactive stent.
FIG. 8 illustrates an example method to prepare a radioactive stent.
FIG. 9 illustrates an example method to prepare a radioactive stent.
FIG. 10 illustrates an example method to prepare a radioactive stent.
FIG. 11 illustrates an example method to prepare a radioactive stent.
FIG. 12 illustrates an example method to prepare a radioactive stent.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications and alternatives falling within the scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

Treatment of abnormal tissue growth (e.g. cancer) may be accomplished through a variety of methodologies. For example, treatment of cancer may include the placement and deployment of a stent across the diseased tissue. However, in some instances stenting outcomes may be improved by combining one or more conventional therapies. For example, combining stent placement with radiation therapy may improve cancer treatment outcomes as compared to either stent or radiation therapy alone. Therefore, it may be desirable to utilize materials and/or design a stent that combines traditional stenting with radiation therapy. Some of the examples and methods disclosed herein may include a stent that can deliver radiation therapy.

Stents disclosed herein may treat esophageal cancers. Additionally, the stent may treat other forms of disease (e.g., cancers), including gastrointestinal, pancreatic, colon, tracheal, urethra, ureter, cardiac, brain, breast, bladder, kyphoplasty and peripheral vascular disease, for example. Further, the stents disclosed herein may also be used in excisional cavities in solid and/or hollow organs.

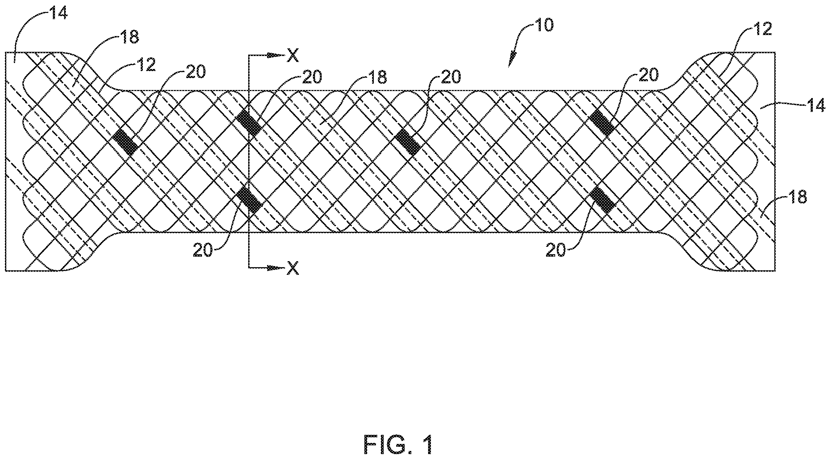

Figure 1 shows an example radioactive stent 10. Stent 10 may include a plurality of filaments and/or strut members 12 arranged in a variety of different designs and/or geometric patterns to form an expandable framework. For example, strut members 12 may be laser cut from a unitary tubular member. In other examples, filaments 12 may be braided, woven, knitted or constructed using a combination of these (or similar) manufacturing techniques. Therefore, numerous designs, patterns and/or configurations for the stent cell openings, strut thicknesses, strut designs, stent cell shapes are contemplated and may be utilized with embodiments disclosed herein. Additionally, as shown in FIG. 1, stent 10 may include first and/or second flared end regions.

Further, stent 10 may be delivered to a treatment area via a stent delivery system (not shown). For example, in some instances stent 10 may be a balloon expandable stent. Balloon expandable stents may be manufactured from a single, cylindrical tubular member (e.g., a cylindrical tubular member may be laser cut to form an expandable stent 10).

In other instances stent 10 may be a self-expanding stent. A self-expanding stent may be delivered to a treatment area via a self-expanding stent delivery system. It is contemplated that the examples disclosed herein may be utilized with any one of various stent configurations, including, balloon expandable stents, such as a laser cut stent and/or a braided stent, a self-expanding stent, non-expandable stents, or other stents.

Figure 1 further shows stent 10 including one or more tubular members 18. Tubular members 18 may include one or more of a variety of radioactive elements coupled and/or attached thereto. The radioactive elements 20 may be separated from each other by one or more spacers (not shown). As will be discussed in greater detail below, tubular members 18 may extend helically along stent 10. Figure 1 shows tubular members 18 extending along the entire length of stent 10 (e.g., extending along both flared end regions in addition to the stent portion between the flared end regions). However, in other examples, the tubular members 18 may extend only along a portion of stent 10.

In some instances, stent 10 may be a self-expanding stent. Self-expanding stent examples may include an expandable framework having one or more filaments combined to form a rigid and/or semi-rigid stent structure. For example, stent filaments 12 may be braided, intertwined, interwoven, weaved, knitted or the like to form an expandable frame. Self-expanding stents may be manufactured from a single, cylindrical tubular laser-cut Nitinol members.

Stent 10 (including stent filaments 12 and/or tubular members 18) in examples disclosed herein may be constructed from a variety of materials. For example, stent 10 (e.g. self-expanding or balloon expandable) may be constructed from a metal (e.g., Nitinol). In other instances, stent 10 may be constructed from a polymeric material (e.g., PET). In yet other instances, stent 10 may be constructed from a combination of metallic and polymeric materials. Additionally, stent 10 may include a bioabsorbable and/or biodegradable material.

Stent 10 includes a covering 14. For example, filaments 12 and/or tubular members 18 may be partially or fully covered by an elastomeric or non-elastomeric material. Additionally, filaments 12 and/or tubular members 18 may be partially or fully covered by a polymeric material such as silicone or ePTFE. Further, the covering 14 (e.g., polymer) may span the spaces (e.g., openings, cells) in the wall of stent 10. As will be discussed in greater detail below, the covering 14 may be applied by spraying, dipping, spinning or attaching a polymer sheet or tube to the inner and/or outer surface of stent 10. The covering 14 covers both the stent filaments 12 and the tubular members 18. Further, in some examples, the covering 14 may cover a combination of one or more of the stent filaments 12 and one or more of the tubular members 18. For example, in some examples covering 14 may be configured to attach the tubular members 18 to filaments 12.

In at least one example, it is contemplated that filaments 12 (of stent 10) may be designed to contain radioactive material. For example, Figure 1 shows one or more filaments 12 including a hollow portion designed to carry (e.g., hold, contain, etc.) radioactive material such as a radioactive seed 20. In some examples, the center of one or more of filaments 12 may be hollow along the entire length of the filament. Further, it is contemplated that filaments 12 could be pre-loaded with radioactive material (e.g., seed 20) prior to being formed (e.g., braided, wound, etc.) into stent member 10. In other examples, hollow filaments 12 could be loaded with radioactive material (e.g., seed 20) just prior to stent 10 being delivered to a target site.

Radioactive seeds 20 may include a variety of radioactive materials and or combinations of various materials. For example, seed 20 may include Iodine-125 (e.g. GE Oncura THINSeed™, IsoAid Advantage™ by IsoAid, Best™ Iodine-125), Palladium-103 (e.g. CivaString™ by CivaTech Technology, Theraseed™ by Theragenics, Best™ Palladium-103), Cesium-131, Gold-198, Iridium-192 and/or Ytterbium-169 or any other variations and/or derivatives thereof. Further, seed 20 may include other types of radioactive material. Additionally, seed 20 may include beta-emitting radionuclides.

In general, seeds 20 may be positioned adjacent a target site, whereby seeds 20 may release radioactive energy and/or material, thereby radioactively treating the target location. Seed 20 may be generally shaped as shown in Figure 1. In other words, seed 20 may be an elongated cylinder. However, other shapes are contemplated. For example, seeds 20 may be rounded, ovular, rectangular, triangular, or the like. Seeds 20 may be spaced and/or distributed in various patterns and/or distributions along tubular members 18. For example, seeds 20 may be spaced apart from one another via spacers. In other words, any number of spacers may be positioned in between seeds 20 to create a particular distribution, spacing and/or arrangement of seeds 20 along tubular members 18. The distribution and/or arrangement seeds 20 in combination with spacers may vary depending on particular design considerations.

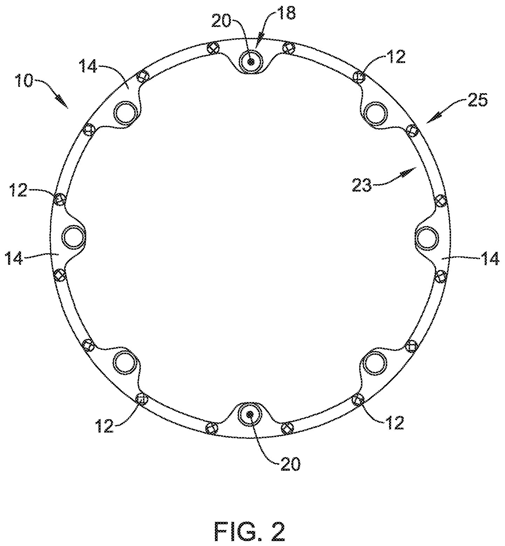

Figure 2 illustrates a cross-section along line X-X of the example stent 10 shown in Figure 1. As shown in Figure 2, covering 14 may contact the stent filaments 12, tubular members 18 or both the stent filaments 12 and the tubular members 18. Further, in some examples, the covering 14 may cover a combination of one or more of the stent filaments 12 and one or more of the tubular members 18. For example, in some examples covering 14 may be configured to attach the tubular members 18 to filaments 12. For example, in at least some examples disclosed herein, coating 14 may be directly applied to the filaments 12 (defining an expandable framework) and/or tubular members 18. Further, in some examples, coating 14 may be utilized to bind, attach, couple or tie filaments 12 (e.g., the expandable framework) to tubular members 18. In some examples, coating 14 may resemble a web or matrix structure that includes both the expandable framework 12 and the tubular members 18.

Further, in some examples expandable framework 12 and/or tubular members 18 may be encased and/or encapsulated in coating 14. In other words, in some examples, coating 14 may substantially surround all surfaces of expandable framework 12 and/or tubular members 18. However, in other instances, tubular members 18 and/or expandable framework 12 (including filaments 12) may be embedded in coating 14. For purposes of this disclosure, the term embedded may be defined as describing instances in which tubular members 18 and/or expandable framework 12 (including filaments 12) are either partially or fully surrounded by coating 14. In other words, in some examples, tubular members 18 and/or expandable framework 12 may be embedded in coating 14 such that a portion of the tubular member 18 and/or expandable framework 12 extends above and/or away from coating 14 (e.g., a portion of the tubular member may be free of coating 14) while an adjacent portion of the tubular member 18 and/or expandable framework 12 remains attached to coating 14.

Additionally, stent 10 may include an inner surface 23 and an outer surface 25. In some examples, such as that shown in Figure 2, tubular members 18 may be attached to filaments 12 along the inner surface 23 of stent 10. Additionally, as discussed above, one or more of tubular members 18 may include one or more radioactive seeds 20 located within the tubular member 18 (e.g., within a hollow portion of the tubular members 18).

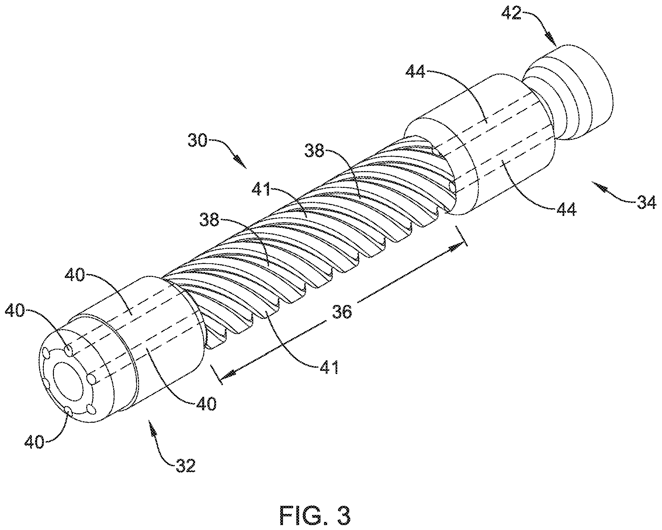

An example process for preparing a radioactive stent as described above will be described with respect to Figures 3-10. Figure 3 illustrates an example mandrel 30 designed to position tubular members 18 and filaments 12 relative to one another prior to the application of covering 14. As shown in Figure 3, mandrel 30 may include a first end portion 32, a second end portion 34 and a middle (e.g., body) portion 36. Body 36 may include one or more channels and/or grooves 38 extending in a helical orientation around the outer surface of mandrel 30. While Figure 3 shows grooves 38 extending helically around mandrel 30, it is contemplated that grooves 38 may be configured in a variety of other arrangements/orientations around mandrel 30. For example, grooves 38 may extend straight (e.g., parallel to one another) along the longitudinal axis of body 36.

It is contemplated that mandrel 30 may be designed to include one or more grooves 38 extending around the outer surface of mandrel 30. For example, it is contemplated that groove 38 may be a single, continuous channel extending helically along mandrel 30. However, in other examples, grooves 38 may include 2, 3, 4, 5, 6, 7, 8, 9, 10 or more separate grooves wound in parallel to one another. For example, Figure 3 shows six individual grooves 38 wound in parallel with one another.

Additionally, in some instances it is contemplated that mandrel 30 may include two or more grooves 38 which are helically wound in the same direction. Further, mandrel 30 may only include grooves 38 which are wound in the same direction. In other words, mandrel 30 may be free of helical grooves which are wound in directions opposite to one another.

However, in other instances it is contemplated that mandrel 30 may include two or more grooves 38 which are helically wound in opposite directions. For example, mandrel 30 may include two or more grooves 38, each configured as a helix which are wound opposite to one another.

Further, mandrel 30 may include two or more helical grooves 38 wound in the same direction and spaced substantially equidistant from one another. Additionally, the grooves 38 may include a continuous raised land 41 positioned between the two or more grooves. The continuous raised land may extend along the length of the body 36 from the first end portion 32 to the second end portion 34. In other instances, the continuous raised land 41 may extend the entire length of mandrel 30 (e.g., in instances where grooves 38 extend the entire length of mandrel 30).

As will be described in greater detail below, first end portion 32 and/or second end portion 34 may include a first securement region and a second securement region, respectively. The securement regions may define a variety of structures that aid in positioning tubular members 18 prior to the application of coating 14. In other words, the securement regions may secure a portion of the tubular members 18 (e.g., one or both of the ends of a tubular member 18) to mandrel 30 prior to the application of a coating 14.

A variety of securement mechanisms are contemplated in addition to the securement mechanisms described in examples below. For example, in some instances, first end portion 32 and/or second end portion 34 may include clips, posts, couplers, fasteners, hooks, channels, grooves adhesives or any combination thereof to secure tubular members 18 to mandrel 30.

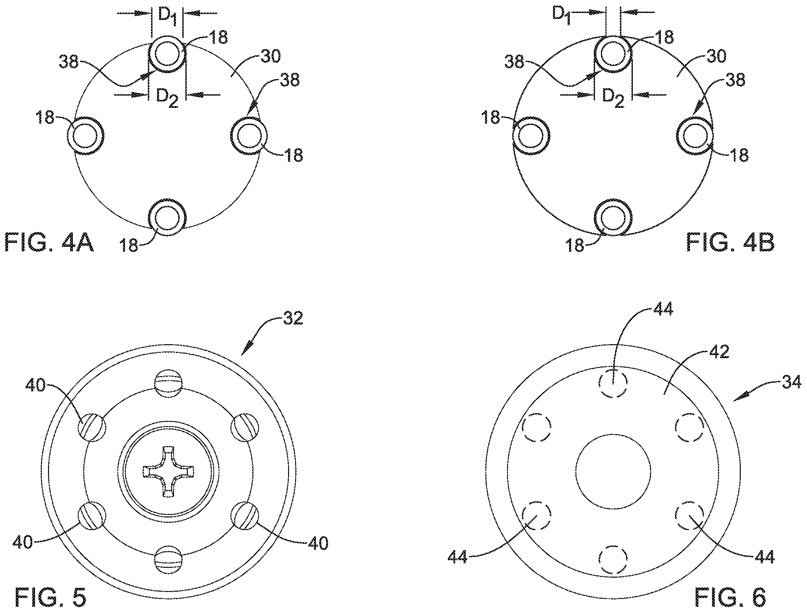

For example, Figure 4A illustrates a cross-section of mandrel 30 showing grooves 38 and example tubular members 18 positioned within grooves 38. It can be appreciated that the cross-section illustrated in Figure 4A may be a cross-section taken along any portion of mandrel 30. For example, the securement mechanism illustrated in Figure 4A may represent grooves 38 on the body 36 of mandrel 30 and/or first end portion 32 and/or second end portion 34.

Figure 4A illustrates an example securement mechanism whereby tubular members 18 may be positioned within grooves 38 such that a portion of tubular members 18 extend beyond (e.g., away from) the outer surface of mandrel 30 while a portion of tubular members 18 remains securely positioned within grooves 38. For example, Figure 4A shows a diameter D1 which defines the size of an opening that groove 38 creates in the outer surface of mandrel 30. Further, Figure 4A illustrates diameter D2 which defines the diameter of a tubular member 18 positioned partially within groove 38. As shown in Figure 4A, it can be appreciated that because D2 is less than D1, tubular member 18 is secured within grooves 38.

Figure 4B illustrates an example securement mechanism whereby tubular members 18 may be positioned within grooves 38 such that a portion of tubular members 18 does not extend beyond the outer surface of mandrel 30. In other words, Figure 4B illustrates an example in which tubular members 18 are positioned fully radially inward from the outer surface of mandrel 30. It can be appreciated that positioning tubular members 18 inward of the outer surface of mandrel 30 may allow for tubular members 18 to be completely covered and/or embedded within a covering applied to the outer surface of mandrel 30. It is contemplated that grooves 38 may have a diameter which results in tubular members 18 being radially inward of the outer surface of mandrel 30, flush with the outer surface of mandrel 30 or extending radially away from the outer surface of mandrel 30.

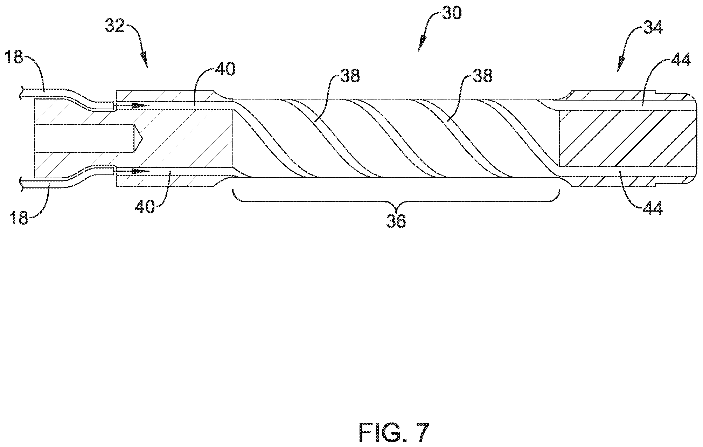

In another example, Figure 3 illustrates a plurality of first end channels 40 extending through first end portion 32. First end channels 40 may be an extension of the grooves 38 extending along body 36. In other words, distal end channels may align in a one-to-one relationship with each of the individual grooves 38. As will be described in more detail with respect to Figure 7, in one method of preparing stent 10, tubular members 18 may be advanced through first end channels 40 prior to being advanced along grooves 38. In other words, distal end channels may guide, align and/or feed tubular members 18 into individual grooves 38.

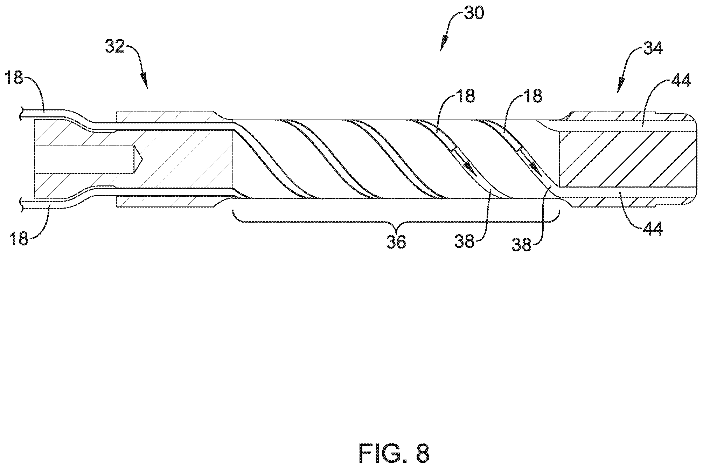

Additionally, Figure 3 shows a plurality of second end channels 44 extending through second end portion 34. Similar to distal end channels 38 discussed above, second end channels 44 may be an extension of grooves 38 extending along body 36. In other words, second end channels 44 may align in a one-to-one relationship with each of the individual grooves 38. As will be described in more detail with respect to Figure 8, in one method of preparing stent 10, tubular members 18 may be advanced through first end channels 40 after to being advanced along grooves 38.

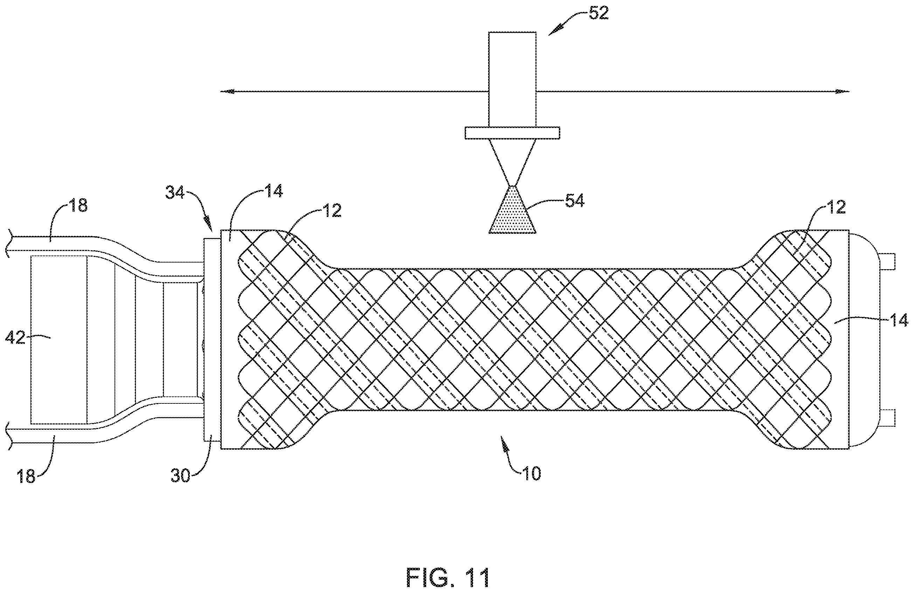

Figure 3 further illustrates an attachment member 42 extending away from second end portion 34. Attachment member 42 may be utilized to hold or grasp mandrel 30 during a manufacturing process. For example, as will be described in greater detail with respect to Figure 11, attachment member 42 may be utilized in a dip coating process.

Figure 5 illustrates an end view of the first end portion 32 of mandrel 30, including first end channels 40. As discussed above, even though Figure 5 shows mandrel 30 including six first end channels 40, it is contemplated that more or less than six distal end channels may be included. As shown in Figure 5, first end channels 40 may extend through first end portion 32. In other words, the first end channels 40 may be defined as apertures and/or lumens that extend through first end portion 32.

Figure 6 illustrates an end view of the second end portion 34 of mandrel 30, including second end channels 44. As discussed above, even though Figure 6 shows mandrel 30 including six second end channels 44, it is contemplated that more or less than six second end channels 44 may be included. As shown in Figure 6, second end channels 44 may extend through second end portion 34. In other words, the second end channels 44 may be defined as apertures and/or lumens that extend through second end portion 34.

Figures 7-9 illustrate one example method for preparing stent 10. As shown in Figure 7, an example first step in preparing stent 10 may include advancing tubular members 18 through first end channels 40 located within first end portion 32. The direction of which tubular members 18 are advanced is depicted by the arrows shown in Figure 7. Figure 7 further illustrates that first end channels 40 may be advanced from the outer surface of first end portion 32, through first end portion 32 and align with individual grooves 38 of body 36.

It can be appreciated that grooves 38 may be sized to accept tubular members 18. For example, Figure 8 illustrates a second step in preparing stent 10 may include advancing tubular members 18 along grooves 38 located in body 36. As discussed above and shown in Figure 8, grooves 38 may be helically oriented around the outer surface of the mandrel 30. Figure 8 further illustrates that tubular members have been advanced through the first end channels 40 which are aligned with grooves 38. Figure 8 further illustrates that tubular members 18 may be advanced along helically-oriented grooves 38 from first end portion 32 toward second end portion 34 (as depicted by the arrows shown in Figure 8). It can further be appreciated that the size of grooves 38 may allow a portion of tubular members 18 to extend above the continuous raised land 41 (shown in Figure 3) of mandrel 30. In other examples, the outer surface of tubular members 18 may remain flush with the outer surface of the mandrel 30 or slightly below.

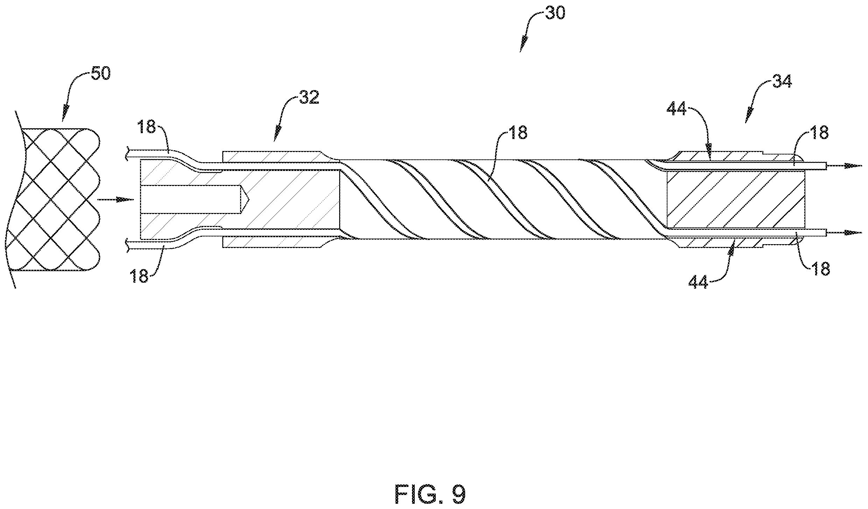

Figure 9 illustrates that a third step in preparing stent 10 may include advancing an expandable framework 50 (e.g., a stent formed from filaments 12) over mandrel 30 (which also includes tubular members 18 positioned along the outer surface thereof). It should be noted that Figure 9 further shows that tubular members have been advanced through the second end channels 44 and are shown extending away from the second end portion 34. At this step in preparing stent 10, one or both of the ends of tubular members 18 may extend and be secured within first end channels 40 and second end channels 44. However, it is also contemplated that tubular members 18 may extend through first and second end channels 40/44 and not be secured within first and second end channels 40/44 prior to advancing expandable framework 50 over tubular members 18 secured to mandrel 30. Additionally, it is contemplated that in some examples, expandable framework 50 may be advanced over mandrel 30 before tubular members 18 are advanced through and secure within second end channels 44.

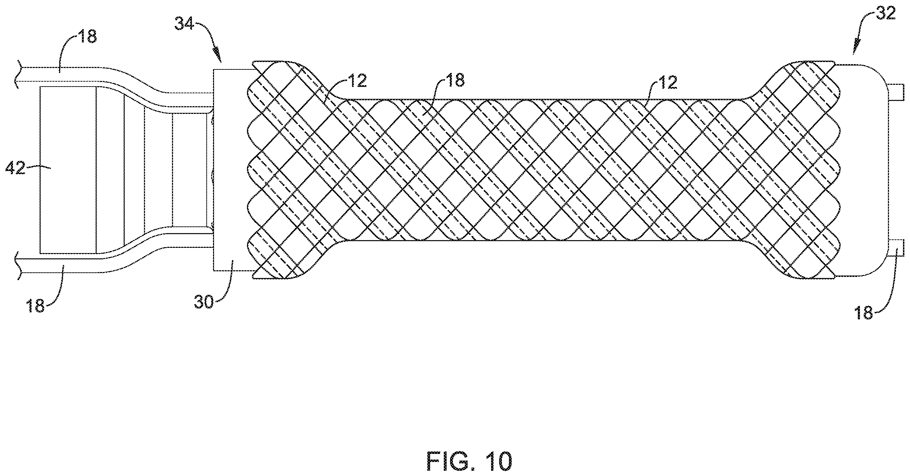

Figure 10 illustrates expandable framework 50 (e.g., a stent formed from filaments 12) positioned over tubular member 18 which have been advanced along the grooves 38 (through distal and proximal end channels) as described above. As shown in Figure 10, a portion of the stent member is positioned over first end portion 32 and second end portion 34. It can be appreciated that first end portion 32 and second end portion 34 may correspond to flared end portions of the stent 10 shown in Figure 1. Additionally, as described above, tubular members 18 are shown extending away from first end portion 32 and second end portion 34.

Figure 11 illustrates an example method form applying covering 14 (described with respect to Figure 1) to both expandable framework 50 (including filaments 12) and tubular members 18. Figure 11 shows spraying element 52 applying a spray 54 to filaments 12 and tubular members 18 positioned on mandrel 30. It can be appreciated that spray 54 may pass through the cells between filaments 12, which may allow spray 54 to contact tubular members 18. For example, as discussed above, spray 54 may form a layer of material on both the inner and outer surfaces of filaments 12, between the cells of filaments 12 and along all or a portion of tubular members 18. In some examples, coating 14 may span the interstitial spaces between cells of expandable framework 50.

As discussed above, spray 54 may correspond to coating 14 which is applied to all or a portion of tubular members 18 and/or expandable framework 50. For example, it can be appreciated that because expandable framework 50 is positioned over tubular members 18, the coating 14 may attach the tubular members 18 to the inner surface of expandable framework 50. Further, as shown in Figure 11, spraying element 52 may translate the full length of mandrel 30, thereby depositing material to all or a portion of expandable framework 50 and/or tubular members 18 accordingly. It is further contemplated that mandrel 30, expandable framework 50 and/or tubular members 18 may be rotated as spray 54 is applied.

The coating 14 may be applied (e.g., disposed) along the outer surface of the expandable framework 50. In other instances it may be favorable to ensure that the coating 14 is positioned on the inner surface of expandable framework 50. In yet other examples, it may be desirable to dispose coating 14 along a combination of the outer surface and inner surface of expandable framework 50. In some instances, the coating 14 may be applied to a surface of the tubular members 18, such as an outer surface and/or an inner surface of the tubular members 18. Additionally, in some instances it may be desirable to apply the coating 14 to all surfaces of expandable framework 50 and/or the tubular members 18. Further, it is also contemplated that any portion of expandable framework 50 and/or tubular members 18 may be "masked" so that a portion of one or more structural characteristics (e.g., filaments 12 and/or tubular members 18) may be left unaltered or be altered to a lesser extent.

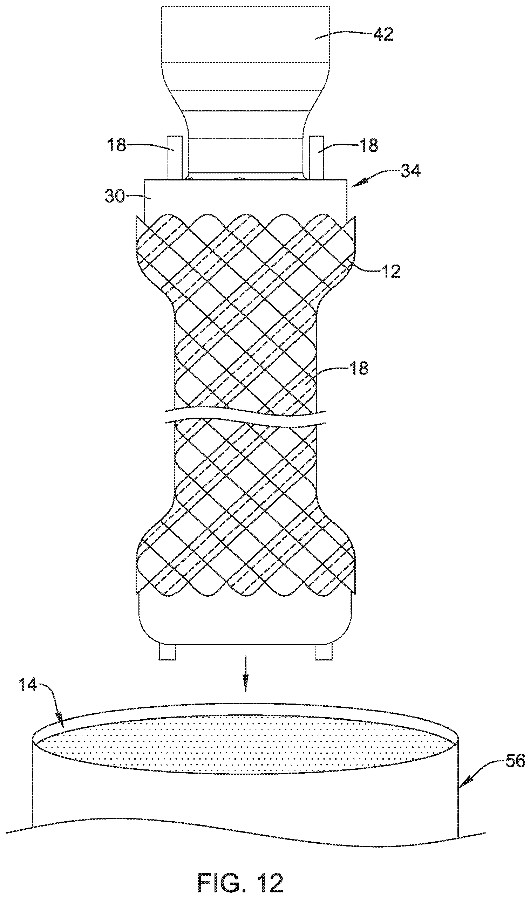

Figure 12 illustrates another example process for preparing stent 10. Figure 12 is an illustration of utilizing a dip coating process to apply coating 14 to a stent 10. As shown in Figure 12, the stent 10 (including expandable framework 50 and/or tubular members 18) may be dipped (e.g., lowered) into the reservoir 56 to apply a coating 14 to the stent 10. The reservoir 56 may include coating 14.

In at least some embodiments, dip coating the stent 10 with coating 14 may occur while moving the stent 10 in and out of the reservoir 56 and/or rotating the stent 10 within the reservoir 56. For example, the stent 10 may be coated by bringing the stent 10 into and out of the reservoir 56, rotating the stent 10 in the reservoir 56, or both. In some instances, however, rotation or translation may not be required. In some embodiments, the speed at which the stent 10 is translated and/or rotated may vary. In general, the rate of motion, duration of time in the reservoir 56 and/or cycles of submerging the stent 10 in the coating 14 may correlate to the amount of coating 14 applied to stent 10. Further, it is also contemplated that any portion of the filaments 12 and/or tubular members 18 may be "masked" so that a portion of one or more structural characteristics (e.g., filaments 12 and/or tubular members 18) may be left unaltered or be altered to a lesser extent.

While examples disclosed herein may illustrate tubular members 18 being positioned on the inner surface of expandable framework 50 and along the body 36 of mandrel 30, it is contemplated that tubular members 18 may be positioned along any portion of the expandable framework 50 and may extend to the ends of mandrel 30. For example, it is contemplated that in some instances mandrel 30 may include grooves 38 which extend onto the surface of first end portion 32 and/or second end portion 34.

In some examples it may be beneficial to design mandrel 30 in multiple component parts which can be separated from one another. For example, in some instances, first end portion 32, second end portion 34 and body 36 may separate into three individual pieces. The individual pieces may then be attached (e.g., via a screw) to form mandrel 30 as disclosed herein.

Materials that may be used for the various components of the stent 10 and the various examples disclosed herein may include those commonly associated with medical devices. For simplicity purposes, the disclosure makes reference to a stent 10. However, this is not intended to limit the devices and methods described herein, as the discussion may be applied to other similar systems and/or components of stent systems or devices disclosed herein.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A stent, comprising:
an expandable framework;
a plurality of tubular members disposed along the expandable framework, each of the plurality of tubular members including a lumen extending therein;
**characterized by**
a coating applied directly to both the plurality of tubular members and the expandable framework.

2. The stent of claim 1, wherein the expandable framework includes one or more interstitial spaces, and wherein the coating is configured to span both the one or more interstitial spaces and the plurality of tubular members.

3. The stent of any one of claims 1-2, wherein the coating attaches the plurality of tubular members to the expandable framework.

4. The stent of any one of claims 1-3, wherein the plurality of tubular members and the expandable framework are embedded in the coating.

5. The stent of any one of claims 1-4, wherein the expandable framework includes an inner surface, and wherein the plurality of tubular members are attached to the inner surface of the expandable framework.

6. The stent of claim 5, wherein the plurality of tubular members are helically wound around the inner surface of the expandable framework.

7. The stent of any one of claims 1-6, further comprising a plurality of radioactive elements disposed within the lumen of one or more of the plurality of tubular members.

8. The stent of claim 7, further comprising one or more spacers positioned between two or more of the plurality of radioactive elements.

9. A method of preparing a stent, the method comprising:
positioning a plurality of tubular members on a mandrel, each of the plurality of tubular members including a lumen extending therein;
disposing an expandable framework over the tubular members and the mandrel; and
applying a coating to both the expandable framework and the tubular members.

10. The method of claim 9, wherein positioning the plurality of tubular members on a mandrel includes winding the plurality of tubular members along grooves located on the surface of the mandrel.

11. The method of claim 10, wherein the plurality of tubular members are helically wound around the surface of the mandrel.

12. The method of any one of claims 9-10, wherein the expandable framework includes one or more interstitial spaces, and wherein the coating is configured to span both the one or more interstitial spaces and the tubular members.

13. The method of any one of claims 9- 12, wherein the coating attaches the tubular members to the expandable framework.

14. The method of any one of claims 9-13, wherein the expandable framework includes an inner surface, and wherein the plurality of tubular members are attached to the inner surface of the expandable framework.

15. The method of any one of claims 9-14, further comprising positioning a plurality of radioactive elements within the lumen of one or more of the plurality of tubular members.

## Patentansprüche

1. Stent, der aufweist:
ein expandierbares Gerüst;
mehrere Röhrenteile, die entlang des expandierbaren Gerüsts angeordnet sind, wobei jedes der mehreren Röhrenteile ein sich darin erstreckendes Lumen aufweist;
**gekennzeichnet durch** eine Beschichtung, die sowohl auf die mehreren Röhrenteile als auch auf das expandierbare Gerüst direkt aufgetragen ist.

2. Stent nach Anspruch 1, wobei das expandierbare Gerüst einen oder mehrere Zwischenräume aufweist und wobei die Beschichtung so konfiguriert ist, dass sie sowohl den einen oder die mehreren Zwischenräume als auch die mehreren Röhrenteile überspannt.

3. Stent nach Anspruch 1 oder 2, wobei die Beschichtung die mehreren Röhrenteile am expandierbaren Gerüst befestigt.

4. Stent nach einem der Ansprüche 1 bis 3, wobei die mehreren Röhrenteile und das expandierbare Gerüst in der Beschichtung eingebettet sind.

5. Stent nach einem der Ansprüche 1 bis 4, wobei das expandierbare Gerüst eine Innenfläche aufweist und wobei die mehreren Röhrenteile an der Innenfläche des expandierbaren Gerüsts befestigt sind.

6. Stent nach Anspruch 5, wobei die mehreren Röhrenteile um die Innenfläche des expandierbaren Gerüsts schraubenförmig gewickelt sind.

7. Stent nach einem der Ansprüche 1 bis 6, ferner mit mehreren radioaktiven Elementen, die im Lumen eines oder mehrerer der mehreren Röhrenteile angeordnet sind.

8. Stent nach Anspruch 7, ferner mit einem oder mehreren Abstandshaltern, die zwischen zwei oder mehr der mehreren radioaktiven Elemente positioniert sind.

9. Verfahren zur Herstellung eines Stents, wobei das Verfahren aufweist:
Positionieren mehrerer Röhrenteile auf einem Dorn, wobei jedes der mehreren Röhrenteile ein sich darin erstreckendes Lumen aufweist;
Anordnen eines expandierbaren Gerüsts über den Röhrenteilen und dem Dorn; und
Auftragen einer Beschichtung sowohl auf das expandierbare Gerüst als auch auf die Röhrenteile.

10. Verfahren nach Anspruch 9, wobei das Positionieren der mehreren Röhrenteile auf einem Dorn aufweist: Wickeln der mehreren Röhrenteile entlang von Nuten, die auf der Oberfläche des Doms liegen.

11. Verfahren nach Anspruch 10, wobei die mehreren Röhrenteile um die Oberfläche des Dorns schraubenförmig gewickelt werden.

12. Verfahren nach Anspruch 9 oder 10, wobei das expandierbare Gerüst einen oder mehrere Zwischenräume aufweist und wobei die Beschichtung so konfiguriert ist, dass sie sowohl den einen oder die mehreren Zwischenräume als auch die Röhrenteile überspannt.

13. Verfahren nach einem der Ansprüche 9 bis 12, wobei die Beschichtung die Röhrenteile am expandierbaren Gerüst befestigt.

14. Verfahren nach einem der Ansprüche 9 bis 13, wobei das expandierbare Gerüst eine Innenfläche aufweist und wobei die mehreren Röhrenteile an der Innenfläche des expandierbaren Gerüsts befestigt werden.

15. Verfahren nach einem der Ansprüche 9 bis 14, das ferner aufweist: Positionieren mehrerer radioaktiver Elemente im Lumen eines oder mehrerer der mehreren Röhrenteile.

## Revendications

1. Endoprothèse vasculaire comprenant :
un bâti expansible ;
une pluralité d'éléments tubulaires disposés le long du bâti expansible, chacun de la pluralité d'éléments tubulaires comprenant une lumière s'étendant à l'intérieur de ce dernier ;
**caractérisée par** :
un revêtement appliqué directement à la fois sur la pluralité d'éléments tubulaires et sur le bâti expansible.

2. Endoprothèse vasculaire selon la revendication 1, dans laquelle le bâti expansible comprend un ou plusieurs espaces interstitiels, et dans laquelle le revêtement est configuré pour couvrir à la fois les un ou plusieurs espaces interstitiels et la pluralité d'éléments tubulaires.

3. Endoprothèse vasculaire selon l'une quelconque des revendications 1 à 2, dans laquelle le revêtement fixe la pluralité d'éléments tubulaires au bâti expansible.

4. Endoprothèse vasculaire selon l'une quelconque des revendications 1 à 3, dans laquelle la pluralité d'éléments tubulaires et le bâti expansible sont noyés dans le revêtement.

5. Endoprothèse vasculaire selon l'une quelconque des revendications 1 à 4, dans laquelle le bâti expansible comprend une surface interne, et dans laquelle la pluralité d'éléments tubulaires sont fixés sur la surface interne du bâti expansible.

6. Endoprothèse vasculaire selon la revendication 5, dans laquelle la pluralité d'éléments tubulaires sont enroulés de manière hélicoïdale autour de la surface interne du bâti expansible.

7. Endoprothèse vasculaire selon l'une quelconque des revendications 1 à 6, comprenant en outre une pluralité d'éléments radioactifs disposés à l'intérieur de la lumière d'un ou de plusieurs de la pluralité d'éléments tubulaires.

8. Endoprothèse vasculaire selon la revendication 7, comprenant en outre un ou plusieurs dispositifs d'espacement positionnés entre deux ou plusieurs de la pluralité d'éléments radioactifs.

9. Procédé pour préparer une endoprothèse vasculaire, le procédé comprenant les étapes suivantes :
positionner une pluralité d'éléments tubulaires sur un mandrin, chacun de la pluralité d'éléments tubulaires comprenant une lumière s'étendant à l'intérieur de ce dernier ;
disposer un bâti expansible sur les éléments tubulaires et le mandrin ;
appliquer un revêtement à la fois sur le bâti expansible et les éléments tubulaires.

10. Procédé selon la revendication 9, dans lequel le positionnement de la pluralité d'éléments tubulaires sur un mandrin comprend l'enroulement de la pluralité d'éléments tubulaires le long de rainures positionnées sur la surface du mandrin.

11. Procédé selon la revendication 10, dans lequel la pluralité d'éléments tubulaires sont enroulés de manière hélicoïdale autour de la surface du mandrin.

12. Procédé selon l'une quelconque des revendications 9 à 10, dans lequel le bâti expansible comprend un ou plusieurs espaces interstitiels, et dans lequel le revêtement est configuré pour couvrir à la fois les un ou plusieurs espaces interstitiels et les éléments tubulaires.

13. Procédé selon l'une quelconque des revendications 9 à 12, dans lequel le revêtement fixe les éléments tubulaires au bâti expansible.

14. Procédé selon l'une quelconque des revendications 9 à 13, dans lequel le bâti expansible comprend une surface interne, et dans lequel la pluralité d'éléments tubulaires sont fixés sur la surface interne du bâti expansible.

15. Procédé selon l'une quelconque des revendications 9 à 14, comprenant en outre l'étape pour positionner une pluralité d'éléments radioactifs à l'intérieur de la lumière d'un ou de plusieurs de la pluralité d'éléments tubulaires.
